# EUROPEAN PATENT SPECIFICATION

(11) **EP 3 220 998 B1**
(45) Date of publication and mention of the grant of the patent: **09.10.2019**
(21) Application number: 15790058.0
(22) Date of filing: 26.10.2015
(51) Int. Cl.: A61M 39/22

(54) **OPENING DEVICE FOR ACTING ONTO A CLOSURE ELEMENT OF A MEDICAL TUBING**
ÖFFNUNGSVORRICHTUNG ZUM EINWIRKEN AUF EIN SCHLIESSELEMENT EINES MEDIZINISCHEN SCHLAUCHS
DISPOSITIF D'OUVERTURE POUR AGIR SUR UN ÉLÉMENT DE FERMETURE D'UNE TUBULURE MÉDICALE

(30) Priority: 17.11.2014 EP 14193459
(43) Date of publication of application: 27.09.2017
(73) Proprietor: Fresenius Kabi Deutschland GmbH, 61352 Bad Homburg (DE)
(72) Inventor: BRÜCKNER, Thomas, 63776 Mömbris (DE); BIEHL, Martin, 66606 St. Wendel (DE)
(74) Representative: Maikowski & Ninnemann Patentanwälte Partnerschaft mbB
(86) International application number: PCT/EP2015/074730
(87) International publication number: WO 2016/078872

(56) References cited:
- WO-A1-01/85231
- US-A1- 2003 212 381
- US-A1- 2012 216 891

## Description

The invention relates to an opening device for acting onto a closure element of a medical tubing according to the preamble of claim 1.

An opening device of this kind, commonly referred to also as "breaking device" or "breaker", comprises a housing and an opening mechanism arranged in or on the housing. The opening mechanism is constituted to act onto a closure element of a tubing for opening a flowpath within the tubing. A drive arrangement is provided for mechanically or electro-motorically driving the opening mechanism.

In medical tubing sets, for example within a blood bag system comprising one or multiple blood bags or other liquid containers, closure elements are placed within tubing sections in order to close a flowpath through the corresponding tubing. By closing one or multiple tubing sections of a tubing set, a blood bag system can for example be stored or transported. In order to prepare a blood component, for example for infusion purposes, a flowpath through a tubing from one container to another may then be opened acting onto the closure element within a tubing for opening a corresponding flowpath.

Different systems of closure elements exist for closing a tubing and, hence, preventing a flow through the tubing. Closure elements generally have the shape of a cap or pin which is insertable into the lumen of a tubing such that, in an inserted state, the lumen of the tubing is closed off and a flow through the tubing is prevented. The different existing systems herein differ in the way the closure elements can be opened in order to allow a flow through the tubing.

In a first system, as for example described in WO 2004/058046 A2, which discloses the combination of features of the preamble of claim 1, or WO 2012/080664 A2, an opening of a flowpath can be achieved by breaking the closure element. In another system, as described for example in WO 2006/114319 A1, an opening of a flowpath can be achieved by deforming the closure element without actually breaking it. In both cases, the closure element even after opening remains in the tubing, but is broken or deformed such that a flow through the tubing is no longer prevented.

In principle, the opening of a closure element of this kind can be achieved manually by manually breaking or deforming the closure element within the tubing. For example, a closure element as described in WO 2012/080664 A2 can be broken by manually grabbing a tubing at the location of the closure element and by repeatedly bending it until the closure element breaks.

However, this may be a tedious task for a user such that there is a desire to provide an automatic opening device which can automatically open a closure element within a tubing.

Herein, because different systems of closure elements exist, one opening device may not be suitable to open different kinds of closure elements, because the different closure elements may require a different opening action. This may make it necessary to provide different opening devices for different closure elements, which however is costly and therefore to be avoided. Instead of using multiple different opening devices, an opening device may comprise different adapter plates, the adapter plates having different configurations such that they are suitable to be used in connection with different closure elements, as for example described in WO 2014/083412 A1. This, however, makes it necessary to replace one adapter plate by another when using the opening device with different closure elements, which again may be tedious for a user.

It is an object of the instant invention to provide an opening device which in an easy-to-use manner allows for opening closure elements of different kinds.

This object is achieved by means of an opening device comprising the features of claim 1.

Accordingly, the opening mechanism comprises a first tube reception device and a second tube reception device, the first reception device and the second tube reception device each being constituted to receive a medical tubing for acting onto a closure element of the tubing, wherein the opening device is displaceable relative to the housing between a first position in which a tubing is insertable into the first tube reception device and a second position in which a tubing is insertable into the second tube reception device.

By providing a displaceable opening mechanism the opening device is adapted to act on different tubings and closure elements arranged therein. Namely, the opening mechanism is adapted to receive different kinds of tubings in different tube reception devices such that different closure elements can be treated differently for opening a flowpath within a corresponding tubing.

The opening device, hence, can selectively act on different tubings and closure elements arranged therein. In order to act on a closure element of a first kind the opening mechanism of the opening device can be brought into the first position relative to the housing, whereas in order to act on a closure element of a second kind the opening mechanism can be brought into a second position. The opening device can be adjusted for receiving a tubing in the first tube reception device or in the second tube reception device by displacing the opening mechanism relative to the housing such that in the first position of the opening mechanism a tubing can be inserted into the first tube reception device, and in the second position of the opening mechanism another tubing can be inserted into the second tube reception device.

In order to act on different closure elements in different tubing sections, the opening mechanism comprises different breaker arrangements. Herein, a first breaker arrangement forms the first tube reception device and a second breaker arrangement forms the second tube reception device. Each breaker arrangement is constituted to act onto a closure element of a tubing for opening a flowpath within a tubing.

Because different closure elements may require a different mechanical movement action to open them, the breaker arrangements preferably are constituted to perform different breaking actions for breaking different closure elements. In this regard, the term "breaking" is understood in a general sense in that it refers to a general opening of a closure element. The opening of a closure element not necessarily involves an actual breaking, i.e., a mechanical separation of parts of the closure element, but may take place by physically deforming a closure element without actually breaking it.

The breaking actions of the breaker arrangements beneficially are adapted such that they effectively can open different closure elements. Herein, a closure element of a first kind may require a pressing action in a transverse direction relative to a longitudinal extension direction of a tubing in order to deform a closure element located within an inner lumen of the tubing. A second type of a closure element, in contrast, may require a physical breaking, i.e., an actual separation of parts, of the closure element. This may require a repeated bending of the tubing and the closure element arranged therein to break off a part of the closure element.

The first breaker arrangement may, for example, comprise a first breaking element and a second breaking element which together form the first tube reception device into which a tubing together with a closure element arranged therein can be inserted. The breaking elements may, for example, be constituted such they can be laterally moved with respect to each other to act onto a closure element within a tubing in a transverse direction perpendicular to a longitudinal direction of extension of the tubing. The first breaker arrangement, hence, is adapted to perform a pressing action for deforming the closure element within the tubing.

For example, the tubing together with the closure element may be inserted into the first tube reception device in-between the breaking elements of the first breaker arrangement, and by pressing the breaking elements towards each other a closure element within the tubing may be deformed in order to open a flowpath within the tubing.

The second breaker arrangement, in contrast, may comprise a first breaking element and a second breaking element which are pivotable with respect to each other. The first breaking element and the second breaking element together form the second tube reception device into which a tubing and together with it a closure element contained in the tubing may be inserted. By pivoting the breaking elements with respect to each other the tubing can be bent back and forth in a repeated fashion such that a closure element in the tubing is subject to a breaking force until eventually a part of the closure element breaks off and a flowpath within the tubing is opened.

The opening mechanism, hence, comprises different breaker arrangements adapted to receive a tubing and constituted to perform different breaking actions to open different closure elements. The opening device herein may be adapted to use the first breaker arrangement or the second breaker arrangement by displacing the opening mechanism within the housing such that in the first position of the opening mechanism the first breaker arrangement can be used, whereas in the second position of the opening mechanism the second breaker arrangement can be employed.

In one embodiment, the breaker arrangements are actuated by a first actuation element and a second actuation element of the opening mechanism. The first and the second actuation element for example are laterally movable with respect to each other for actuating the first and/or the second breaker arrangement. The actuation elements can engage with the drive arrangement such that via the drive arrangement the actuation elements can be actuated for driving the first and/or the second breaker arrangement.

In one embodiment, the first breaking element of the first breaker arrangement is rigidly arranged on a first actuation element, whereas the second breaking element of the first breaker arrangement is rigidly connected to the second actuation element. The breaking elements of the first breaker arrangement may for example be integrally formed with the respective actuation elements such that the breaking elements arranged on the actuation elements can be moved by laterally displacing the actuation elements with respect to each other for performing a pressing action onto a tubing received in-between the breaking elements of the first breaker arrangement.

In addition, the first and the second actuation element may also serve to drive the second breaker arrangement. For this, the first actuation element and the second actuation element, in one embodiment, are mechanically coupled to the second breaker arrangement such that a lateral movement of one or both of the actuation elements induces a pivoting movement of the breaking elements of the second breaker arrangement with respect to each other.

In one embodiment, the first breaking element of the second breaker arrangement may, for example, be rotatably arranged on the first actuation element, whereas the second breaking element of the second breaker arrangement is pivotably connected to the first breaking element. By additionally coupling the first breaking element of the second breaker arrangement to the second actuation element and the second breaking element to the first actuation element, a relative movement of the actuation elements with respect to each other will cause a pivoting movement of the breaking elements of the second breaker arrangement with respect to each other such that a tubing received on the breaking elements can be bent back and forth in a repeated fashion for breaking a closure element arranged in the tubing.

The actuation elements serve to transfer a driving movement of the drive arrangement to the first or the second breaker arrangement for actuating the first or the second breaker arrangement. The drive arrangement, for example, can comprise a drive element which is rotatable about a rotational axis and engages the first and/or the second actuation element such that a rotational movement of the drive element causes a lateral movement of the first actuation element, the second actuation element or both.

The engaging coupling of the drive element with the first and/or the second actuation element herein may depend on the position of the opening mechanism within the housing. In a first coupling state, a rotational movement of the drive element may, for example, be transferred into an opposing lateral movement of the actuation elements with respect to each other. The relative position of the actuation elements with respect to each other, hence, can be adjusted for example in order to approach the breaking elements arranged on the actuation elements towards each other for performing a pressing action onto a tubing received in-between the breaking elements. In a second coupling state, in contrast, the first and the second actuation element may be moved together in a lateral direction for laterally displacing the opening mechanism as a whole within the housing. In the second coupling state, hence, the drive element drives the opening mechanism to displace it between its first position and its second position in order to bring the first or the second breaker arrangement into a position in which it can receive a tubing. In a third coupling state, the drive element may again act onto the first and second actuation element for inducing a relative, opposing lateral movement of the actuation elements with respect to each other, for example for driving the second breaker arrangement, i.e., for pivoting the breaking elements of the second breaker arrangement with respect to each other for acting onto a tubing arranged in-between the breaking elements of the second breaker arrangement.

In one particular embodiment, the drive element may be constituted to act onto the opening mechanism in the first position of the opening mechanism for actuating the first breaker arrangement to act onto a tubing in the first reception device. Hence, by driving the drive element in the first position of the opening mechanism, the actuation elements can be moved relative to each other for approaching breaking elements arranged on the actuation elements towards each other. The drive element may further be constituted to act onto the opening mechanism in order to displace it between the first and the second position by moving the first and the second actuation element together in a common lateral displacement direction. And, in the second position of the opening mechanism, the drive element may be constituted to act onto the actuation elements for actuating the second breaker arrangement for performing a pivoting action of its breaking elements.

In one embodiment, the drive element may engage the first actuation element, but not the second actuation element, for displacing the opening mechanism between the first and the second position. Hence, for laterally displacing the opening mechanism within the housing to transfer the opening mechanism from its first position to its second position or vice a versa, the drive element acts onto the first actuation element and moves it laterally. Herein, the second actuation element is carried along without the drive element directly acting onto it. The drive element transfers an adjustment force, hence, onto the first actuation element, and with the lateral movement of the first actuation element the second actuation element is carried along in a passive fashion.

In order to ensure that the second actuation element is carried along when displacing the opening mechanism between its first and second positions, a coupling, for example a magnetic coupling, between the first actuation element and the second actuation element may be provided such that the second actuation element is reliably carried along when moving the first actuation element.

For selectively acting onto both the first and the second actuation element, or only onto the first actuation element, the drive element may comprise two pins protruding from a body of the drive element. The two pins protrude from the body along the rotational axis and are displaced with respect to each other transversely to the rotational axis. Herein, the two pins have different lengths (viewed along the rotational axis) such that a first of the two pins having a longer length may engage with both the first and the second actuation element, but a second of the two pins having a shorter length may engage only with the first actuation element. Depending on the rotational position of the drive element, hence, the first, longer pin engages with the second actuation element or not. If the first, longer pin does not engage with the second actuation element, no physical engagement of the drive element with the second actuation element is present, because the second, shorter pin cannot engage with the second actuation element.

The engagement of the drive element with the actuation elements by means of the pins can be achieved, for example, by providing guide openings in the shape of grooves at bottom faces of the actuation elements into which the pins can engage and in which the pins can move. By moving within the guide openings, the rotational movement of the drive element may be transferred into a lateral movement of one or both of the actuation elements.

Preferably, the drive arrangement is constituted to electro-motorically drive the opening mechanism. For this, the drive arrangement may for, for example, comprise an electric motor. However, it may also be possible to use a manual, mechanical drive arrangement by, for example, providing a lever which can be actuated manually by a user.

In one embodiment, the tubing can be inserted into the first tube reception device or in the second tube reception device in an insertion direction, wherein the opening mechanism is displaceable relative to the housing along a displacement direction transverse to the insertion direction. The displacement direction of the opening mechanism, hence, is directed transversely to the insertion direction such that, by transversely displacing the opening mechanism, the opening device can be adapted to receive a tubing in the first tube reception device of the first breaker arrangement or the second tube reception device of the second breaker arrangement.

In this regard it is to be noted that the displacement direction not necessarily extends along a straight longitudinal line, but may be curved. The displacement of the opening mechanism may involve a pivoting of the opening mechanism.

The idea underlying the invention shall subsequently be described in more detail with regard to the embodiments shown in the figures. Herein:
- Fig. 1A: shows an opening device comprising an opening mechanism in a first position for receiving a tubing in a first tube reception device;
- Fig. 1B: shows the opening device with the opening mechanism in a second position for receiving a tubing in a second tube reception device;
- Fig. 2A-2C: show views of the opening mechanism while actuating a first breaker arrangement forming the first tube reception device;
- Fig. 3A-3C: show the arrangement of Fig. 2A-2C in different views;
- Fig. 4A: shows a cross-sectional view of a tubing having a closure element arranged therein, in a closing state of the closure element;
- Fig. 4B: shows a cross-sectional view of the arrangement of Fig. 4A, with the closure element in a deformed state;
- Fig. 5A-5C: show views of the opening mechanism while actuating a second breaker arrangement forming the second tube reception device;
- Fig. 6: shows a perspective bottom view of the opening mechanism;
- Fig. 7: shows a separate view of a drive element for driving actuation elements of the opening mechanism;
- Fig. 8A-8G: show different views while driving the opening mechanism by means of the drive element;
- Fig. 9A, 9B: show different views of the second breaker arrangement together with a second actuation element;
- Fig. 10: shows a separate view of a first actuation element;
- Fig. 11A-11C: show different views of parts of the opening mechanism in a first pivoting state of the second breaker arrangement;
- Fig. 12A-12C: show different views of parts of the opening mechanism in a second pivoting state of the second breaker arrangement; and
- Fig. 13A-13C: show different views of parts of the opening mechanism in a third pivoting state of the second breaker arrangement.

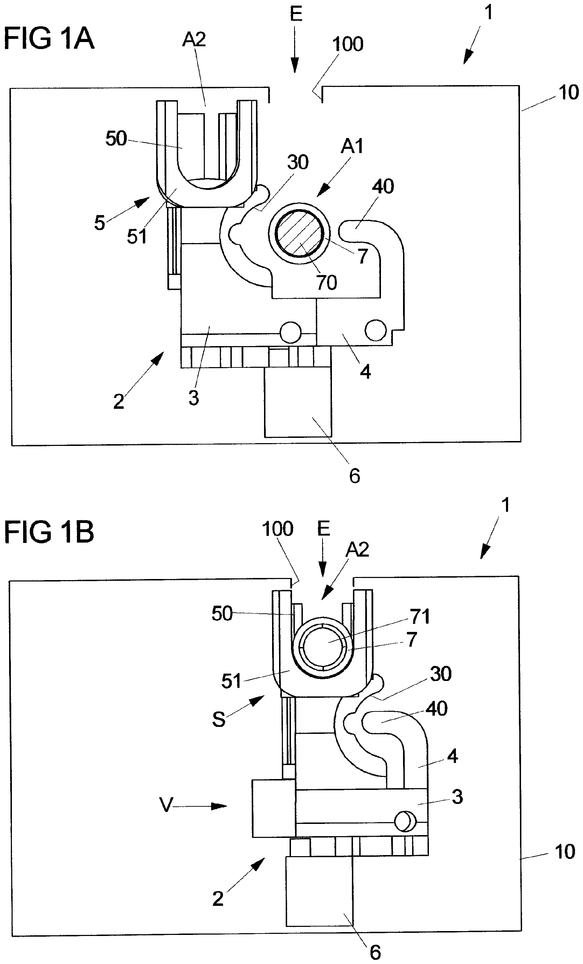

Fig. 1A, 1B to 13A to 13C show an embodiment of an opening device 1 which comprises two breaker arrangements 3, 4; 5 forming two tube reception openings A1, A2, each constituted to receive a tubing 7 therein.

The opening device 1 serves to provide different breaker arrangements 3, 4; 5 for acting onto different closure elements 70, 71 arranged in different tubings 7 for opening a flowpath within the respective tubing 7. By providing two different breaker arrangements 3, 4; 5, a versatile opening device 1 is provided which can treat different closure elements 70, 71 of different tubings 7 in an easy, versatile manner.

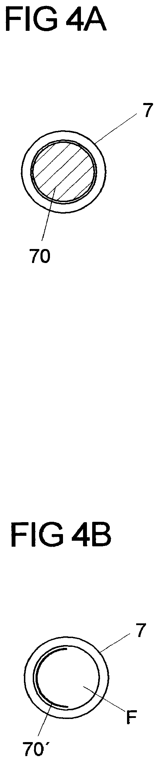

Different closure elements 70, 71 of different tubings 7 may require a different action to deform or break a closure element contained in a tubing 7 in order to open a flowpath through the tubing 7. As shown in Fig. 4A, 4B, a closure element 70 may in a closing state (Fig. 4A) close an inner lumen of a tubing 7 by effectively filling the inner lumen of the tubing 7. The closure element 70, which for example may be of a kind as described in WO 2006/114319 A1, may for example be deformed by pressing it together in a transverse direction such that in a deformed state as shown in Fig. 4B the deformed closure element 70' does no longer fill the inner lumen of the tubing 7, but gives way to a flowpath F through the tubing 7.

Other closure elements 71, for example as described in WO 2012/080664 A2 or WO 2004/058046 A2, may require a different opening action, for example to break off a part of the closure element 71 in order give way to a flowpath F.

Because different closure elements 70, 71 require a different action for opening them (subsequently referred to as "breaking action"), different breaker arrangements 3, 4; 5 are provided within the opening device 1. The opening device 1, as shown in Fig. 1A, 1B, is constituted to receive a tubing 7 in a first tube reception device A1 of a first breaker arrangement 3, 4 or in a second tube reception device A2 of a second breaker arrangement 5. The opening device 1 herein can selectively be adapted to receive a tubing 7 either in the first breaker arrangement 3, 4 or in the second breaker arrangement 5 by displacing an opening mechanism 2 relative to a housing 10.

Herein, in a first position of the opening mechanism 2 (Fig. 1A), the first breaker arrangement 3, 4 with its tube reception device A1 is placed underneath an opening 100 in the housing 10 such that a tubing 7 can be inserted in an insertion direction E through the opening 100 into the tube reception device A1. In a second position, in contrast, the opening mechanism 2 is displaced in a displacing direction V within the housing 10 such that the second tube reception device A2 of the second breaker arrangement 5 is placed underneath the opening 100 of the housing 10 so that a tubing 7 now can be inserted into the second tube reception device A2.

The different breaker arrangements 3, 4; 5 are constituted to perform different breaking actions. In particular, the first breaker arrangement 3, 4 comprises breaking elements 30, 40 in the shape of a semi-circular reception opening 30 and a protruding nose 40 which can be approached towards each other in order to press onto a tubing 7 received in the first tube reception device A1 in-between the breaking elements 30, 40.

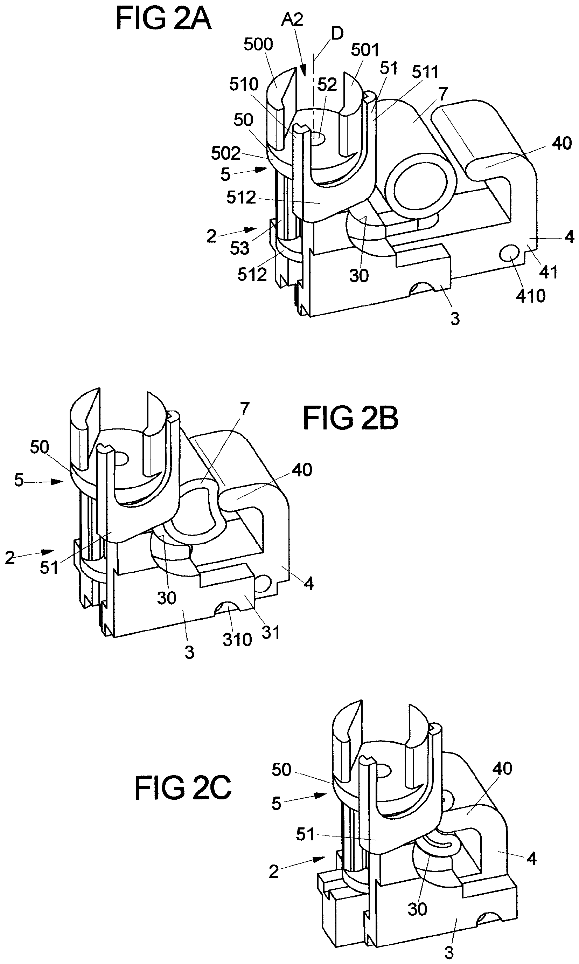

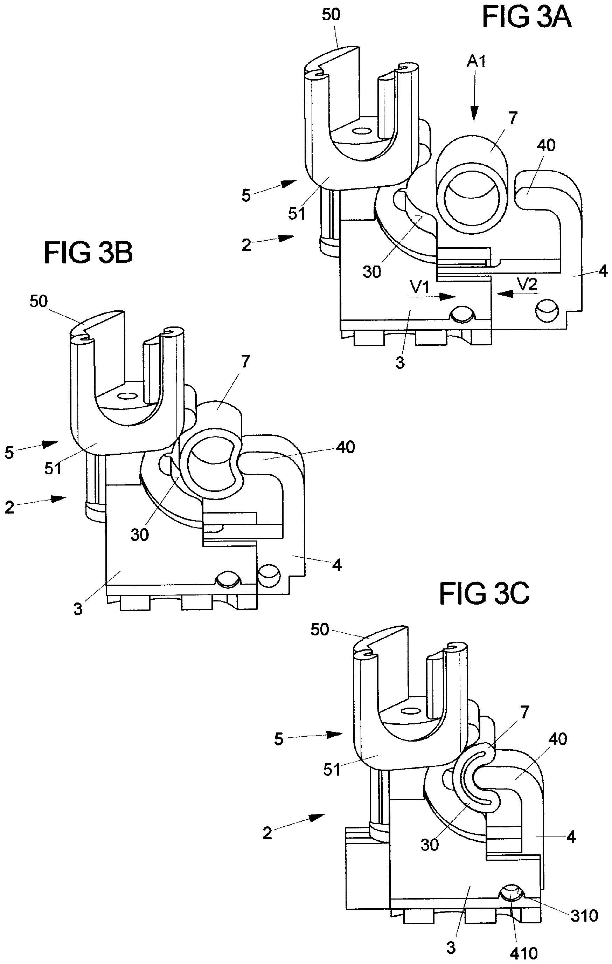

This is shown in Fig. 2A to 2C and 3A to 3C. The breaking elements 30, 40 are integrally formed on actuation elements 3, 4 which, in opposing displacement directions V1, V2, can be moved relative to each other such that the breaking elements 30, 40 can approach each other to deform a tubing 7 received in-between the breaking elements 30, 40. In this way, a closure element 70 can be deformed as shown in Fig. 4A, 4B in order to open up a flowpath F within the tubing 7.

Using the first breaker arrangement 3, 4, typically a single breaking action is sufficient to open the closure element 70 in order to irreversibly deform it as shown in Fig. 4B. Once the tubing 7 has been pressed together in-between the breaking elements 30, 40, the closure element 70 assumes the deformed state of Fig. 4B and remains in this deformed state such that the flowpath F in the tubing 7 is opened. After separating the breaking elements 30, 40 from each other by reversely displacing the actuation elements 3, 4, the tubing 7 can be taken out of the tube reception device A1 and can be used for further processing of blood components or the like.

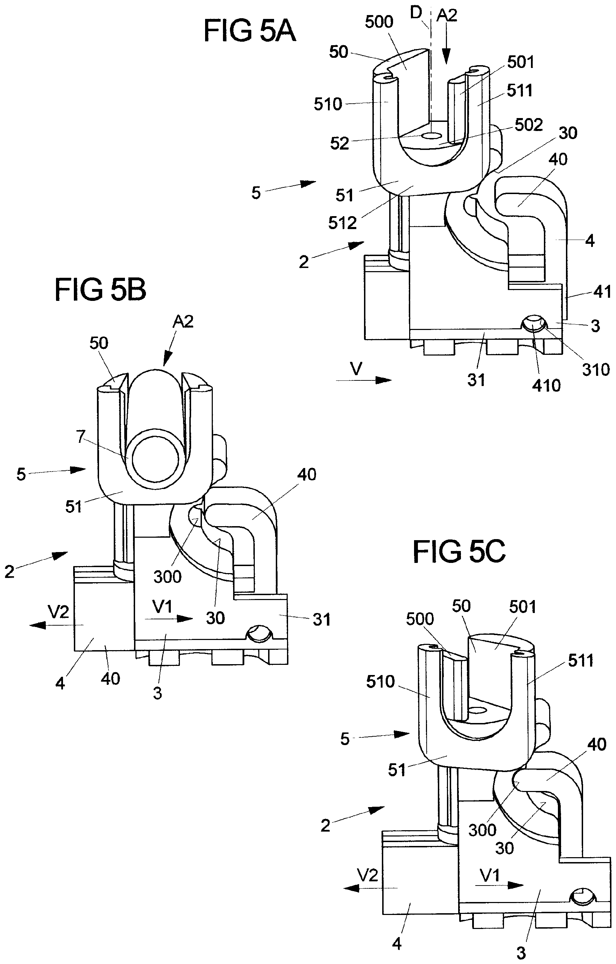

The second breaker arrangement 5 performs a different breaking action. The second breaker arrangement 5 can be brought into an active state by displacing the overall opening mechanism 2 into the second position as shown in Fig. 1B. The second breaker arrangement 5 comprises breaking elements 50, 51 which can be pivoted with respect to each other as it is shown in the different views of Fig. 5A to 5C. The first breaking element 50 comprises protruding flank elements 500, 501 arranged on a common base 502, whereas the second breaking element 51 has a general U-shape having legs 510, 511 connected by a common base 512. With the flank elements 500, 501 and the legs 510, 511 the breaking elements 50, 51 are adapted to receive a tubing 7, wherein the lateral distance in-between the flank elements 500, 501 and the legs 510, 511 is chosen such that it matches the outer diameter of a tubing 7 which shall be received in the tube reception device A2 of the second breaker arrangement 5.

To suitably arrange a tubing 7 within the tube reception device A2 of the second breaker arrangement 5, the breaking elements 50, 51 are preferably brought into a position in which they substantially align as it is shown in Fig. 5B. In this position of the breaking elements 50, 51, a tubing 7 can easily be inserted in the insertion direction E into the tube reception device A2 formed between the flank elements 500, 501 of the first breaking element 50 and the legs 510, 511 of the second breaking element 51.

In order to break a closure element arranged within the inner lumen of the tubing 7, the breaking elements 50, 51 are pivoted with respect to each other, as it is shown in Fig. 5A and 5C. The breaking elements 50, 51 herein can be pivoted back and forth in a repeated fashion such that a part can be broken of the closure element within the tubing 7 to effect an opening of the closure element.

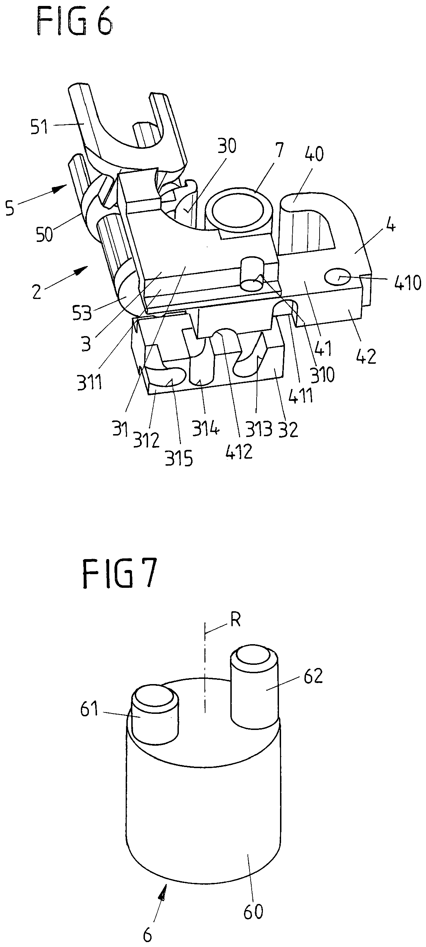

The opening mechanism 2, as it is shown in a separate view in Fig. 6, is driven by a drive element 6, as shown in a separate view in Fig. 7. The drive element 6 is rotatable about a rotational axis R and, for example, is connected with an electric motor to motorically drive the drive element 6.

The drive element 6 comprises a body 60 which is rotationally symmetric about the rotational axis R and can be rotated about the rotational axis R. On the body 60 two pins 61, 62 are arranged, which protrude from the body 60 along the rotational axis R and have different lengths.

By means of the pins 61, 62, the drive element 6, dependent on its rotational position, engages guide openings 313-315 on a bottom face 32 of a first actuation element 3 and guide openings 411, 412 on a bottom face 42 of a base body 41 of a second actuation element 4. The second actuation element 4 herein is guided in-between a first part 311 and second part 312 of a base body 31 of the first actuation element 3 such that it is slidably displaceable in-between the parts 311, 312 of the base body 31 of the first actuation element 3.

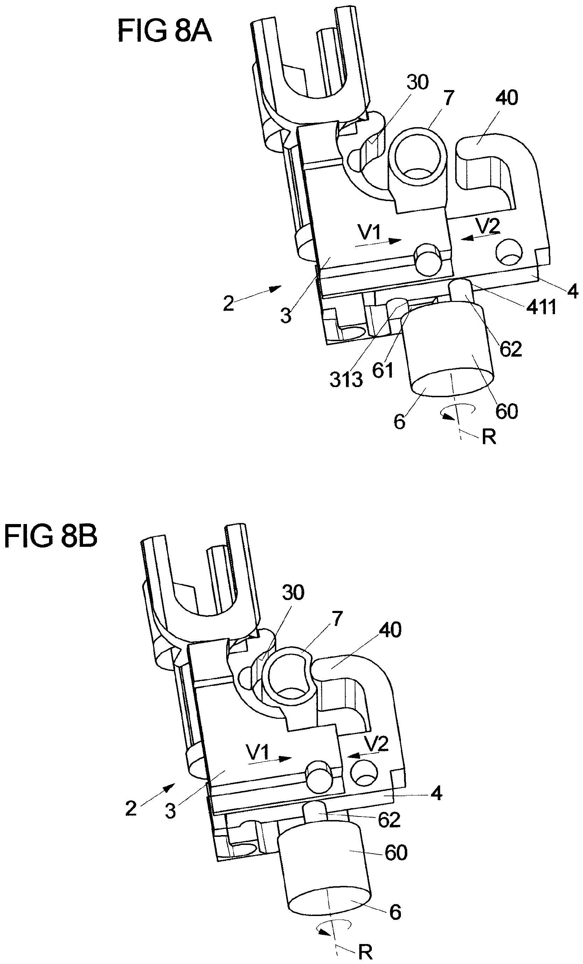

The guide openings 313-315 are formed by indentations on the bottom face 32 of the part 311 of the first actuation element 3. Likewise, the guide openings 411, 412 are formed by indentations on the bottom face 42 of the base body 41 of the second actuation element 4. The bottom faces 32, 42 of the actuation elements 3, 4 herein are located on different heights (when viewed along the rotational axis R of the drive element 6, see for example Fig. 8A), which has the consequence that the longer pin 62 of the drive element 6 can engage both the guide openings 313-315 of the first actuation element 3 and the guide openings 411, 412 of the second actuation element 4, but the other, shorter pin 61 of the drive element 6 can engage only with the guide openings 313-315 of the first actuation element 3 because it does not reach into the guide openings 411, 412 of the second actuation element 4 independent of the rotational state of the drive element 6.

The driving of the opening mechanism 2 by means of the drive element 6 is illustrated in Fig. 8A to 8G.

In Fig. 8A, the opening mechanism 2 is in its first position (Fig. 1A), in which it can receive a tubing 7 in the first tube reception device A1. In this state, in which the breaking elements 30, 40 of the actuation elements 3, 4 are in a position with respect to each other such that a tubing 7 can be inserted in-between the breaking elements 30, 40, the longer pin 62 engages the guide opening 411 of the second actuation element 4, whereas the other, shorter pin 61 engages the guide opening 313 of the first actuation element 3.

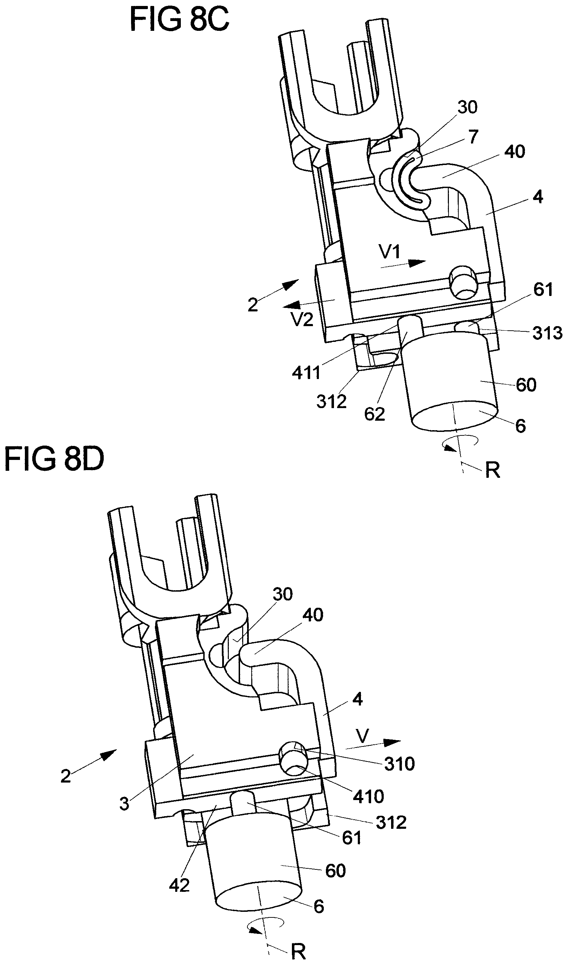

By rotating the drive element 6 in the rotational direction indicated by the arrow in Fig. 8A and 8B, the first actuation element 3 and the second actuation element 4 are moved relative to each other in opposing displacement directions V1, V2 such that the breaking elements 30, 40 are approached towards each other, and the tubing 7 inserted in-between the breaking elements 30, 40 is compressed as shown in Fig. 8C. Because the drive element 6 via its pins 61, 62 engages both the first actuation element 3 and the second actuation element 4, the actuation elements 3, 4 are driven to move in opposing directions V1, V2 until the fully compressed position of Fig. 8C is reached.

In order to remove the tubing 7 from the breaker arrangement 3, 4, the breaking elements 30, 40 can be separated from one another by driving the drive element 6 in the opposite rotational direction, such that the actuation elements 3, 4 are reversed to the initial state of Fig. 8A.

If - without a tubing 7 inserted into the tube reception device A1 - the actuation elements 3, 4 are driven towards the position of Fig. 8C and beyond the position of Fig. 8C, the longer pin 62 disengages the guide opening 411 of the second actuation element 4 and enters the guide opening 314 of the first actuation element 3 (see Fig. 6). Via the longer pin 62 the drive element 6, hence, engages the first actuation element 3. Meanwhile the other, shorter pin 61 is rotated along the bottom face 42 of the second actuation element 4, but does not engage any guide opening 411, 412 of the second actuation element 4, because it is too short to come into engagement with the second actuation element 4. The drive element 6 hence is coupled only with the first actuation element 3 and consequently drives only the first actuation element 3 when rotated beyond the position of Fig. 8C.

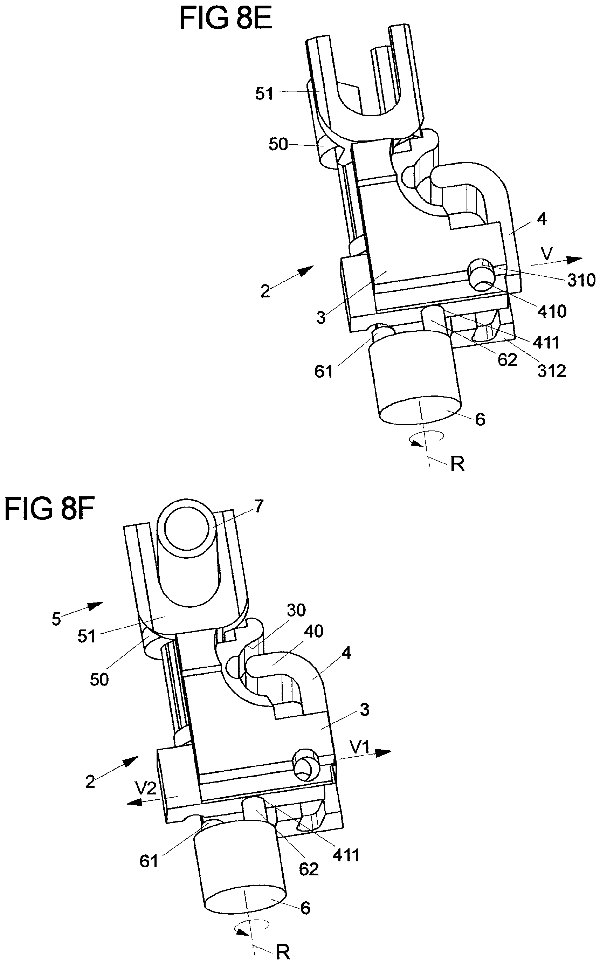

This is indicated in Fig. 8D and 8E. In this state, by rotating the drive element 6 in the direction of the arrow about its rotational axis R, the first actuation element 3 is displaced in the displacement direction V, caused by the coupling with the drive element 6.

Herein, the second actuation element 4 is carried along without actually being coupled to the drive element 6. This is achieved simply because the second actuation element 4 is arranged on the first actuation element 3, such that a displacement movement of the first actuation element 3 causes the second actuation element 4 to be moved together with the first actuation element 3. In addition, magnets may be provided in magnet openings 310, 410 of the first actuation element 3 and the second actuation element 4 such that, in the state of Fig. 8D and 8E, a magnetic coupling between the actuation element 3, 4 is provided ensuring that the second actuation element is carried along when displacing the first actuation element 3 in the displacement direction V.

Once the position of Fig. 8E is reached, the longer pin 62 of the drive element 6 once more comes into engagement with the guide opening 411 of the second actuation element 4. This causes the actuation elements 3, 4, upon further rotation of the drive element 6 in the direction of the arrow in Fig. 8E and 8F, to be once more displaced with respect to each other in opposing displacement directions V1, V2, because the drive element 6 now again is coupled to both actuation elements 3, 4 via the longer pin 62 engaging into the guide opening 411 of the second actuation element 4 and the shorter pin 61 engaging the guide opening 315 of the first actuation element 3.

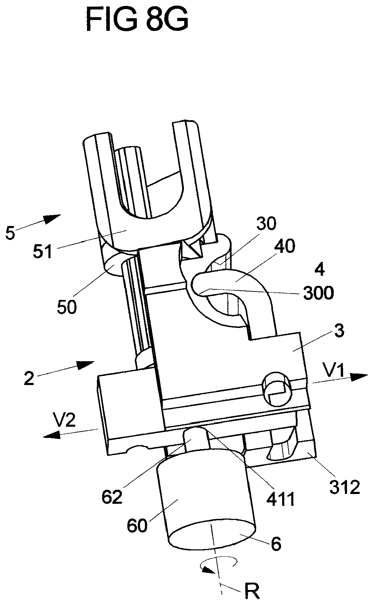

By displacing the actuation elements 3, 4 with respect to each other, as shown in Fig. 8E to 8G, the breaking elements 50, 51 of the second breaker arrangement 5 are caused to pivot with respect to each other. The opening mechanism 2 now is in the second position (Fig. 1B) and may receive a tubing 7 on the breaking elements 50, 51, such that by pivoting the breaking elements 50, 51 with respect to each other the tubing 7 may be bent back and forth for breaking a closure element arranged in the tubing 7. As shown in Fig. 8F and 8G, for pivoting the breaking elements 50, 51 with respect to other, the actuation elements 3, 4 are moved relative to each other such that the breaking elements 30, 40 are further approached towards each other until the position of Fig. 8G is reached in which the nose-like breaking element 40 reaches into an indentation 300 on the semi-circular breaking element 30. This represents a stop position beyond which the breaking elements 50, 51 of the second breaker arrangement 5 cannot be pivoted because the breaking elements 30, 40 of the actuation elements 3, 4 cannot further approach each other.

By repeatedly driving the drive element 6 back and forth between the positions of Fig. 8E and 8G, the second breaker arrangement 5 can repeatedly be actuated for bending the tubing 7 received on the breaking elements 50, 51 back and forth. This allows for breaking off a part of a closure element arranged within the tubing 7.

By means of the opening mechanism 2, a single drive element 6 is used to actuate the first breaker arrangement 3, 4, to displace the opening mechanism 2 from its first position to its second position and vice versa, and to actuate the second breaker arrangement 5. The actuation of the first breaker arrangement 3, 4 herein takes place by displacing the actuation elements 3, 4 with respect to each other in order to approach the breaking elements 30, 40 towards each other. The actuation of the second breaker arrangement 5, in contrast, is caused in that a relative displacement movement of the actuation elements 3, 4 is translated into a pivoting movement of the breaking elements 50, 51.

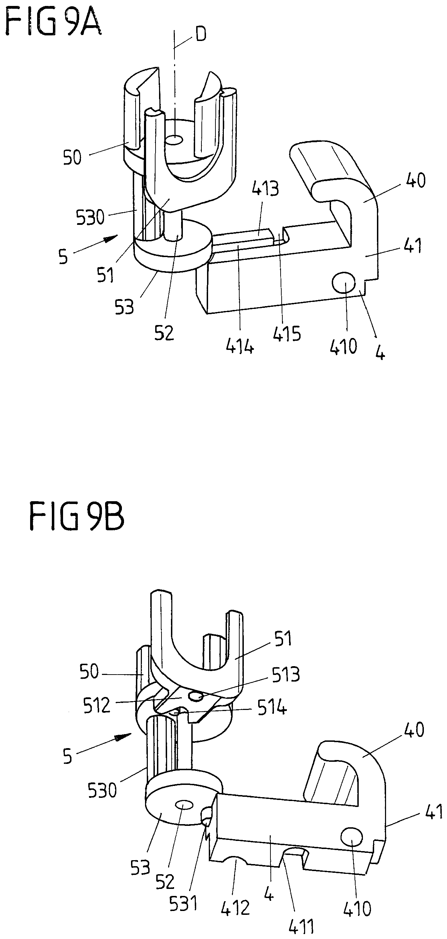

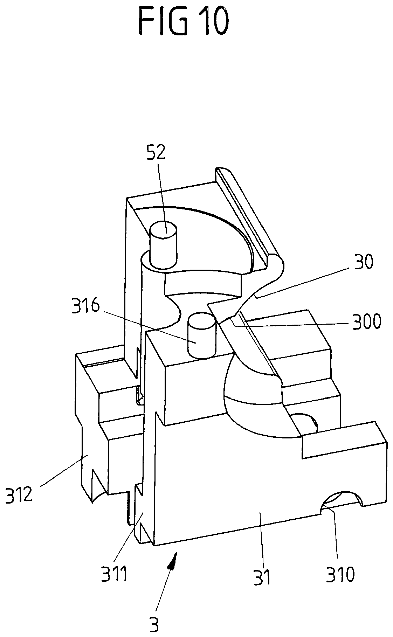

As shown in Fig. 9A and 9B, the first breaking element 50 of the second breaker arrangement 5 is connected with a shaft 52 which is rotatably arranged on the first actuation element 3, as it is shown in Fig. 10. Via the shaft 52, the first breaking element 50, hence, is rotatable about a rotational axis D on the first actuation element 3.

To the first breaking element 50 a disk element 53 is fixedly connected by means of a connection element 530 and in addition by means of the shaft 52 such that the disk element 53 is rotatable about the rotational axis D together with the first breaking element 50. The disk element 53, as shown in Fig. 9B, comprises a pin 531 at its bottom face and via the pin 531 engages a guide opening 413 on the base body 41 of the second actuation element 4.

The second breaking element 51, via a pivoting point 514, is pivotably connected to the first breaking element 50, as it is shown in Fig. 9B. The second breaking element 51 furthermore comprises a coupling opening 530 at its base 512 via which it is coupled with the first actuation element 3 in that a pin 316 (see Fig. 10) of the first actuation element 3 engages into the opening 513 of the second breaking element 51.

The actuation of the second breaker arrangement 5 takes place in the following way.

When the opening mechanism 2 is in the region of its first position (Fig. 1A), the pin 531 on the disc element 53 (see Fig. 9B) is located in a longitudinal section 414 of the guide opening 413, as it is shown in Fig. 9A and 9B. At this position of the pin 531, a displacement movement of the second actuation element 4 does not cause a pivoting movement of the first breaking element 50, because the pin 531 is free to move along the longitudinal section 414 of the guide opening 413.

The actuation of the first breaker arrangement 3, 4 in the first position of the opening mechanism 2, hence, does not lead to a pivoting of the breaking elements 50, 51 of the second breaker arrangement 5 with respect to each other.

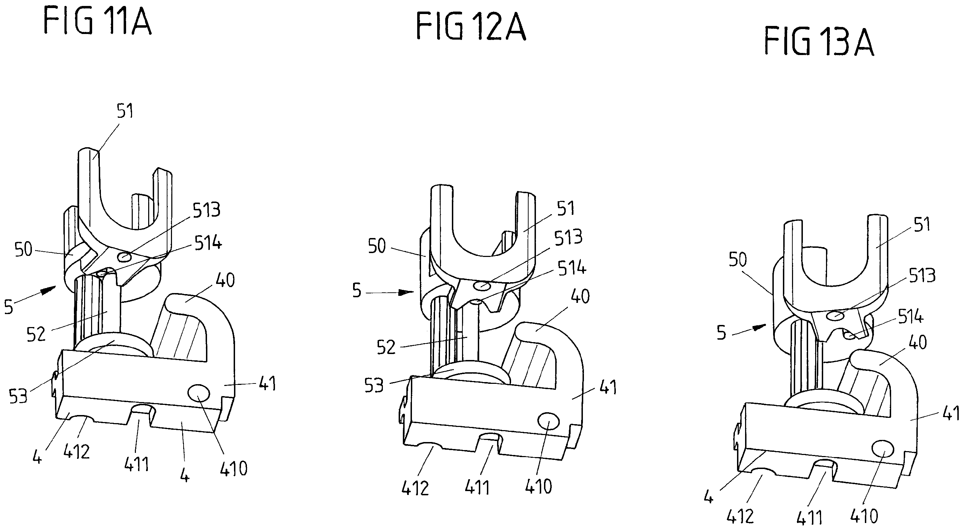

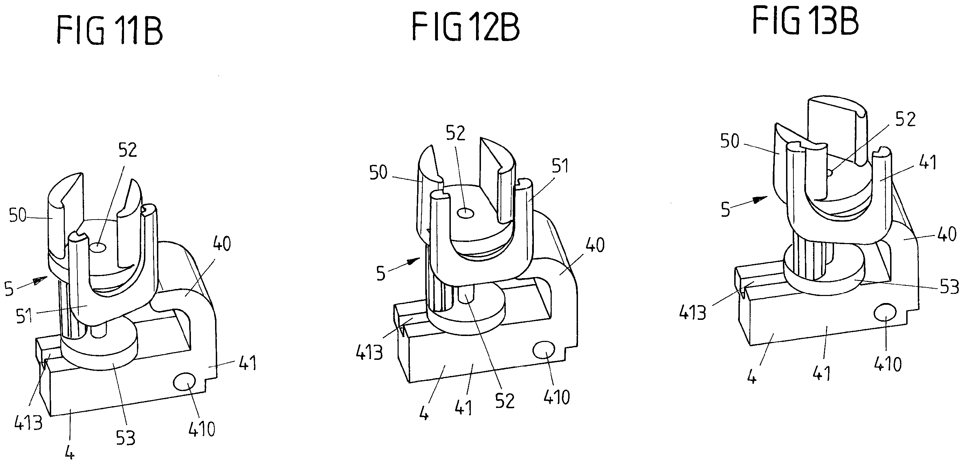

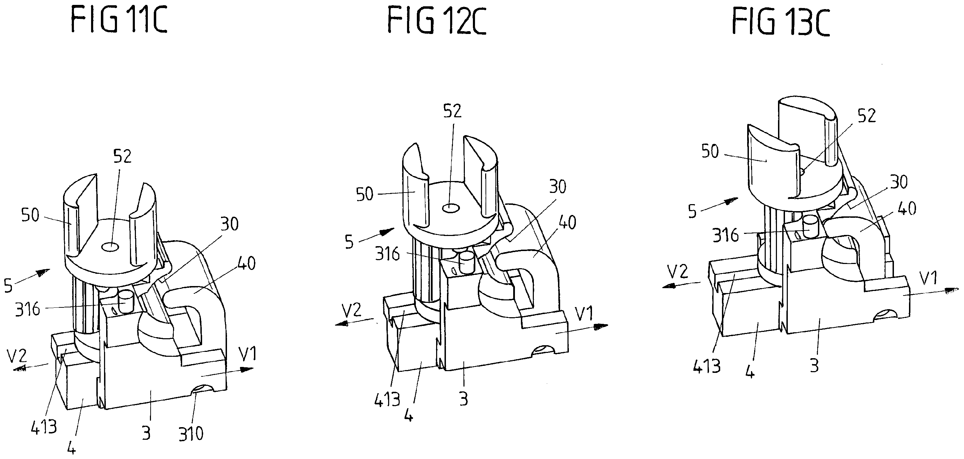

When the opening mechanism 2, however, is displaced towards the second position (Fig. 1B), the pin 531 comes to lie in the transverse section 415 of the guide opening 413. This is illustrated in Fig. 11A-11C to Fig. 13A-13C. If now the actuation elements 3, 4 are moved relative to one another (as it has been described in relation to Fig. 8E to 8G above), the second actuation element 4 acts onto the pin 531 located in the transverse section 415 of the guide opening 413, such that the disk element 53 and together with it the first breaking element 50 is pivoted, as it is shown for example in Fig. 11A, 12A and 13A.

Because the second breaking element 51 is pivotably connected to the first breaking element 15 via the pivoting point 514 and because the shaft 52 as well as the pin 316 of the first actuation element 3 engaging the opening 513 of the second breaking element 51 are fixed with respect to the first actuation element 3, the second breaking element 51 is caused to pivot in an opposite direction with respect to the first breaking element 50 when the first breaking element 50 is pivoted. The breaking elements 50, 51, hence, perform opposing pivoting movements, as it is illustrated in Fig. 11A-11C to Fig. 13A-13C.

A tubing 7 inserted in-between the flank elements 500, 501 of the first breaking element 50 and the legs 510, 511 of the second breaking element 51, hence, is bent back and forth in order to break a closure element arranged within the tubing 7.

By reversely rotating the drive element 6 (starting from the position Fig. 8G towards the position of Fig. 8A), the displacement of the opening mechanism 2 can be reversed such that the opening mechanism 2 is brought back into its first position according to Fig. 1A such that a tubing 7 can once more be inserted into the first tube reception device A1.

The displacement of the opening mechanism 2 between the first position and the second position takes place automatically in an electro-motoric fashion. A user for this can simply press a button or the like in order to cause the opening device 1 to be adapted for using the first breaker arrangement 3, 4 or the second breaker arrangement 5. No replacement of parts is necessary.

The idea underlying the invention is not limited to the embodiment described above, but may be implemented in an entirely different fashion.

By means of an opening device as described herein, a universal, versatile opening device for opening closure elements of different kinds is provided. In principle, even further breaker arrangements, i.e., more than two breaker arrangements, could be provided for adapting the opening device to even more different kinds of closure elements.

It is conceivable to make the opening mechanism replaceable such that different opening mechanisms could be used, for example for adapting the opening device to different kinds of tubings, for example tubings of different diameters, and/or different kinds of closure elements.

### List of Reference Numerals

- 1: Opening device
- 10: Housing
- 100: Opening
- 2: Opening mechanism
- 3: First actuation element
- 30: Breaking element (reception opening)
- 300: Indentation
- 31: Base body
- 310: Magnet opening
- 311,312: Part
- 313-315: Guide opening
- 316: Pin
- 32: Bottom face
- 4: Second actuation element
- 40: Breaking element (breaking nose)
- 41: Base body
- 410: Magnet opening
- 411, 412: Guide opening
- 413: Guide opening
- 414, 415: Section
- 42: Bottom face
- 5: Breaker arrangement
- 50: Breaking element
- 500, 501: Flank element
- 502: Base
- 51: Breaking element
- 510, 511: Leg
- 512: Base
- 513: Opening
- 514: Pivoting point
- 52: Shaft
- 53: Disk element
- 530: Connection element
- 531: Pin
- 6: Drive element
- 60: Body
- 61, 62: Pin
- 7: Tube
- 70, 70': Closure element of first kind
- 71: Closure element of second kind
- A1, A2: Tube reception device
- D: Rotational axis
- E: Insertion direction
- F: Flowpath
- R: Rotational axis
- V, V1, V2: Displacement direction

## Claims

1. Opening device (1) for acting onto a closure element (70, 71) located within an inner lumen of a medical tubing (7), comprising:
- a housing (10),
- an opening mechanism (2) arranged in or on the housing (10) for acting onto a closure element (70, 71) within an inner lumen of a tubing (7) for opening a flowpath (F) within the tubing (7), and
- a drive arrangement (6) for driving the opening mechanism (2),
wherein
the opening mechanism (2) comprises a first breaker arrangement (3, 4) forming a first tube reception device (A1) and a second breaker arrangement (5) forming a second tube reception device (A2), the first tube reception device (A1) and the second tube reception device (A2) being constituted to receive medical tubings (7) comprising different closure elements (70, 71), each breaker arrangement (3, 4, 5) being constituted to act onto a closure element (70, 71) within an inner lumen of a tubing (7) for opening a flowpath (F) within the tubing (7), **characterised in that** the first breaker arrangement (3, 4) and the second breaker arrangement (5) are constituted to perform different breaking actions for breaking the different closure elements (70, 71), wherein the opening mechanism (2) is displaceable relative to the housing (10) between a first position in which a tubing (7) is insertable into the first tube reception device (A1) and a second position in which a tubing (7) is insertable into the second tube reception device (A2).

2. Opening device (1) according to claim 1, **characterized in that** the first breaker arrangement (3, 4) comprises a first breaking element (30) and a second breaking element (40), the first tube reception device (A1) being formed by the first and the second breaking element (30, 40) of the first breaker arrangement (3, 4), wherein at least one of the breaking elements (30, 40) is laterally movable with respect to the other breaking element (30, 40) for acting onto a closure element (70) of a tubing (7) inserted into the first tube reception device (A1).

3. Opening device (1) according to claim 1 or 2, **characterized in that** the second breaker arrangement (5) comprises a first breaking element (50) and a second breaking element (51), the second tube reception device (A2) being formed by the first and the second breaking element (50, 51) of the second breaker arrangement (5), wherein at least one of the first and the second breaking element (50, 51) is pivotable relative to the other breaking element (50, 51) for acting onto a closure element (70) of a tubing (7) inserted into the second tube reception device (A2).

4. Opening device (1) according to one claims 1 to 3, **characterized in that** the opening mechanism (2) comprises a first actuation element (3) and a second actuation element (4), the first and the second actuation element (3, 4) being laterally movable with respect to each other for actuating the first and/or the second breaker arrangement (3, 4, 5), wherein the drive arrangement (6) is engageable with the first and/or the second actuation element (3, 4) for driving the opening mechanism (2).

5. Opening device (1) according to claim 4, **characterized in that** a first breaking element (30) of the first breaker arrangement (3, 4) is rigidly connected to the first actuation element (3), and a second breaking element (40) of the first breaker arrangement (3, 4) is rigidly connected to the second actuation element (4).

6. Opening device (1) according to claim 4 or 5, **characterized in that** the second breaker arrangement (5) is mechanically coupled to the first and/or the second actuation element (3, 4) such that a first breaking element (50) and/or a second breaking element (51) of the second breaker arrangement (5) are pivotable by laterally moving the first and/or the second actuation element (3, 4).

7. Opening device (1) according to claim 6, **characterized in that** the first breaking element (50) of the second breaker arrangement (5) is rotatably arranged on the first actuation element (3), and the second breaking element (51) of the second breaker arrangement (5) is pivotably connected to the first breaking element (50) of the second breaker arrangement (5), wherein in addition the first breaking element (50) of the second breaker arrangement (5) is coupled to the second actuation element (4) and the second breaking element (51) is coupled to the first actuation element (3) such that a relative movement of the first actuation element (3) and the second actuation element (4) with respect to each other causes a pivoting of the first breaking element (50) and the second breaking element (51) of the second breaker arrangement (5) relative to each other.

8. Opening device (1) according to one of claims 4 to 7, **characterized in that** the drive arrangement comprises a drive element (6) being rotatable about a rotational axis (R) and engaging at least one of the first and the second actuation element (3, 4).

9. Opening device (1) according to claim 8, **characterized in that** the drive element (6) is constituted to act onto the opening mechanism (2)
- in the first position of the opening mechanism (2) for actuating the first breaker arrangement (3, 4) to act onto a tubing (7) in the first reception device (A1),
- between the first position and the second position to displace the opening mechanism (2) between the first and the second position, and
- in the second position of the opening mechanism (2) for actuating the second breaker arrangement (5) to act onto a tubing (7) in the second reception device (A2).

10. Opening device (1) according to claim 8 or 9, **characterized in that** the drive element (6) engages the first actuation element (3), but not the second actuation element (4), for displacing the opening mechanism (2) between the first and the second position, wherein the second actuation element (4) is carried along with the first actuation element (3) when moving the first actuation element (3).

11. Opening device (1) according to one of claims 8 to 10, **characterized in that** the drive element (6) comprises two pins (61, 62) protruding from a body (60) of the drive element (6) along the rotational axis (R) and having different lengths when viewed along the rotation axis (R), wherein a first of the two pins (61, 62) has a first length and is constituted to engage both the first and the second actuation element (3, 4), whereas a second of the two pins (61, 62) has a second length smaller than the first length and is constituted to engage only the first actuation element (3).

12. Opening device (1) according to one of the preceding claims, **characterized in that** the drive arrangement (6) is constituted to electromotorically drive the opening mechanism (2).

13. Opening device (1) according to one of the preceding claims, **characterized in that** the tubing (7) is insertable into the first tube reception device (A1) or the second tube reception device (A2) in an insertion direction (E), wherein the opening mechanism (2) is displaceable relative to the housing (10) along a displacement direction (V) transverse to the insertion direction (E).

## Patentansprüche

1. Öffnungsvorrichtung (1) zur Einwirkung auf ein Verschlusselement (70, 71), das in einem Innenlumen eines medizinischen Schlauchs (7) angeordnet ist, umfassend:
- ein Gehäuse (10),
- einen Öffnungsmechanismus (2), der in oder an dem Gehäuse (10) angeordnet ist, zur Einwirkung auf ein Verschlusselement (70, 71) in einem Innenlumen eines Schlauchs (7) zum Öffnen eines Strömungswegs (F) in dem Schlauch (7), und
- eine Antriebsbaugruppe (6) zum Antreiben des Öffnungsmechanismus (2),
wobei
der Öffnungsmechanismus (2) eine erste Brecherbaugruppe (3, 4), die eine erste Schlauchaufnahmevorrichtung (A1) bildet, und eine zweite Brecherbaugruppe (5), die eine zweite Schlauchaufnahmevorrichtung (A2) bildet, umfasst, wobei die erste Schlauchaufnahmevorrichtung (A1) und die zweite Schlauchaufnahmevorrichtung (A2) dafür ausgebildet sind, medizinische Schläuche (7) aufzunehmen, die verschiedene Verschlusselemente (70, 71) umfassen, wobei jede Brecherbaugruppe (3, 4, 5) dafür ausgebildet ist, auf ein Verschlusselement (70, 71) in einem Innenlumen eines Schlauchs (7) einzuwirken, um einen Strömungsweg (F) in dem Schlauch (7) zu öffnen, **dadurch gekennzeichnet, dass**
die erste Brecherbaugruppe (3, 4) und die zweite Brecherbaugruppe (5) dafür ausgebildet sind, verschiedene Brechwirkungen zum Brechen der verschiedenen Verschlusselemente (70, 71) durchzuführen, wobei der Öffnungsmechanismus (2) relativ zu dem Gehäuse (10) zwischen einer ersten Stellung, in der ein Schlauch (7) in die erste Schlauchaufnahmevorrichtung (A1) einführbar ist, und einer zweiten Stellung, in der ein Schlauch (7) in die zweite Schlauchaufnahmevorrichtung (A2) einführbar ist, verschiebbar ist.

2. Öffnungsvorrichtung (1) gemäß Anspruch 1, **dadurch gekennzeichnet, dass** die die erste Brecherbaugruppe (3, 4) ein erstes Brechelement (30) und ein zweites Brechelement (40) umfasst, wobei die erste Schlauchaufnahmevorrichtung (A1) von dem ersten und dem zweiten Brechelement (30, 40) der ersten Brecherbaugruppe (3, 4) gebildet wird, wobei wenigstens eines der Brechelemente (30, 40) relativ zu dem anderen Brechelement (30, 40) seitlich beweglich ist, um auf ein Verschlusselement (70) eines in die erste Schlauchaufnahmevorrichtung (A1) eingeführten Schlauchs (7) einzuwirken.

3. Öffnungsvorrichtung (1) gemäß Anspruch 1 oder 2, **dadurch gekennzeichnet, dass** die die zweite Brecherbaugruppe (5) ein erstes Brechelement (50) und ein zweites Brechelement (51) umfasst, wobei die zweite Schlauchaufnahmevorrichtung (A2) von dem ersten und dem zweiten Brechelement (50, 51) der zweiten Brecherbaugruppe (5) gebildet wird, wobei wenigstens eines von dem ersten und dem zweiten Brechelement (50, 51) relativ zu dem anderen Brechelement (50, 51) schwenkbar ist, um auf ein Verschlusselement (70) eines in die zweite Schlauchaufnahmevorrichtung (A2) eingeführten Schlauchs (7) einzuwirken.

4. Öffnungsvorrichtung (1) gemäß einem der Ansprüche 1 bis 3, **dadurch gekennzeichnet, dass** der Öffnungsmechanismus (2) ein erstes Betätigungselement (3) und ein zweites Betätigungselement (4) umfasst, wobei das erste und das zweite Betätigungselement (3, 4) relativ zueinander seitlich beweglich sind, um die erste und/oder die zweite Brecherbaugruppe (3, 4, 5) zu betätigen, wobei die Antriebsbaugruppe (6) mit dem ersten und/oder dem zweiten Betätigungselement (3, 4) in Eingriff gebracht werden kann, um den Öffnungsmechanismus (2) anzutreiben.

5. Öffnungsvorrichtung (1) gemäß Anspruch 4, **dadurch gekennzeichnet, dass** ein erstes Brechelement (30) der ersten Brecherbaugruppe (3, 4) starr mit dem ersten Betätigungselement (3) verbunden ist und ein zweites Brechelement (40) der ersten Brecherbaugruppe (3, 4) starr mit dem zweiten Betätigungselement (4) verbunden ist.

6. Öffnungsvorrichtung (1) gemäß Anspruch 4 oder 5, **dadurch gekennzeichnet, dass** die zweite Brecherbaugruppe (5) mechanisch mit dem ersten und/oder dem zweiten Betätigungselement (3, 4) gekoppelt ist, so dass ein erstes Brechelement (50) und/oder ein zweites Brechelement (51) der zweiten Brecherbaugruppe (5) durch seitliche Bewegung des ersten und/oder des zweiten Betätigungselements (3, 4) schwenkbar ist.

7. Öffnungsvorrichtung (1) gemäß Anspruch 6, **dadurch gekennzeichnet, dass** das erste Brechelement (50) der zweiten Brecherbaugruppe (5) drehbar an dem ersten Betätigungselement (3) angeordnet ist und das zweite Brechelement (51) der zweiten Brecherbaugruppe (5) schwenkbar mit dem ersten Brechelement (50) der zweiten Brecherbaugruppe (5) verbunden ist, wobei zusätzlich das erste Brechelement (50) der zweiten Brecherbaugruppe (5) an das zweite Betätigungselement (4) gekoppelt ist und das zweite Brechelement (51) an das erste Betätigungselement (3) gekoppelt ist, so dass eine relative Bewegung des ersten Betätigungselements (3) und des zweiten Betätigungselements (4) relativ zueinander Schwenken des ersten Brechelements (50) und des zweiten Brechelements (51) der zweiten Brecherbaugruppe (5) relativ zueinander bewirkt.

8. Öffnungsvorrichtung (1) gemäß einem der Ansprüche 4 bis 7, **dadurch gekennzeichnet, dass** die Antriebsbaugruppe ein Antriebselement (6) umfasst, das um eine Drehachse (R) drehbar ist und mit wenigstens einem von dem ersten und dem zweiten Betätigungselement (3, 4) in Eingriff steht.

9. Öffnungsvorrichtung (1) gemäß Anspruch 8, **dadurch gekennzeichnet, dass** das Antriebselement (6) dafür ausgebildet ist, auf den Öffnungsmechanismus (2) einzuwirken:
- in der ersten Stellung des Öffnungsmechanismus (2), um die erste Brecherbaugruppe (3, 4) zu betätigen, um auf einen Schlauch (7) in der ersten Aufnahmevorrichtung (A1) einzuwirken,
- zwischen der ersten Stellung und der zweiten Stellung, um den Öffnungsmechanismus (2) zwischen der ersten und der zweiten Stellung zu verschieben, und
- in der zweiten Stellung des Öffnungsmechanismus (2), um die zweite Brecherbaugruppe (5) zu betätigen, um auf einen Schlauch (7) in der zweiten Aufnahmevorrichtung (A2) einzuwirken.

10. Öffnungsvorrichtung (1) gemäß Anspruch 8 oder 9, **dadurch gekennzeichnet, dass** das Antriebselement (6) mit dem ersten Betätigungselement (3) aber nicht mit dem zweiten Betätigungselement (4) in Eingriff steht, um den Öffnungsmechanismus (2) zwischen der ersten und der zweiten Stellung zu verschieben, wobei das zweite Betätigungselement (4) mit dem ersten Betätigungselement (3) getragen wird, wenn sich das erste Betätigungselement (3) bewegt.

11. Öffnungsvorrichtung (1) gemäß einem der Ansprüche 8 bis 10, **dadurch gekennzeichnet, dass** das Antriebselement (6) zwei Stifte (61, 62) umfasst, die von einem Körper (60) des Antriebselements (6) entlang der Drehachse (R) vorragen und, wenn entlang der Drehachse (R) gesehen, unterschiedliche Längen aufweisen, wobei ein erster der beiden Stifte (61, 62) eine erste Länge aufweist und dafür ausgebildet ist, sowohl in das erste als auch das zweite Betätigungselement (3, 4) einzugreifen, während ein zweiter der beiden Stifte (61, 62) eine zweite Länge aufweist, die kleiner als die erste Länge ist, und dafür ausgebildet ist, nur in das erste Betätigungselement (3) einzugreifen.

12. Öffnungsvorrichtung (1) gemäß einem der vorstehenden Ansprüche, **dadurch gekennzeichnet, dass** die Antriebsbaugruppe (6) dafür ausgebildet ist, den Öffnungsmechanismus (2) elektromotorisch anzutreiben.

13. Öffnungsvorrichtung (1) gemäß einem der vorstehenden Ansprüche, **dadurch gekennzeichnet, dass** der Schlauch (7) in einer Einführrichtung (E) in die erste Schlauchaufnahmevorrichtung (A1) oder die zweite Schlauchaufnahmevorrichtung (A2) einführbar ist, wobei der Öffnungsmechanismus (2) entlang einer Verschiebungsrichtung (V) quer zu der Einführrichtung (E) relativ zu dem Gehäuse (10) verschiebbar ist.

## Revendications

1. Dispositif d'ouverture (1) pour agir sur un élément de fermeture (70, 71) situé dans une lumière intérieure d'une tubulure médicale (7), comprenant :
- un boîtier (10),
- un mécanisme d'ouverture (2) agencé dans ou sur le boîtier (10) pour agir sur un élément de fermeture (70, 71) dans une lumière intérieure d'une tubulure (7) pour ouvrir un chemin d'écoulement (F) dans la tubulure (7), et
- un dispositif d'entraînement (6) pour entraîner le mécanisme d'ouverture (2),
le mécanisme d'ouverture (2) comprenant un premier agencement rupteur (3, 4) formant un premier dispositif de réception de tubulure (A1), et un second agencement rupteur (5) formant un second dispositif de réception de tubulure (A2), le premier dispositif de réception de tubulure (A1) et le second dispositif de réception de tubulure (A2) étant constitués pour recevoir des tubulures médicales (7) comprenant différents éléments de fermeture (70, 71), chaque agencement rupteur (3, 4, 5) étant constitué pour agir sur un élément de fermeture (70, 71) dans une lumière intérieure d'une tubulure (7) pour ouvrir un chemin d'écoulement (F) dans la tubulure (7),
**caractérisé en ce que** le premier agencement rupteur (3, 4) et le second agencement rupteur (5) sont constitués pour réaliser différentes actions de rupture pour rompre les différents éléments de fermeture (70, 71), le mécanisme d'ouverture (2) étant déplaçable par rapport au boîtier (10) entre une première position dans laquelle une tubulure (7) est insérable dans le premier dispositif de réception de tubulure (A1) et une seconde position dans laquelle une tubulure (7) est insérable dans le second dispositif de réception de tubulure (A2).

2. Dispositif d'ouverture (1) selon la revendication 1, **caractérisé en ce que** le premier agencement rupteur (3, 4) comprend un premier élément de rupture (30) et un second élément de rupture (40), le premier dispositif de réception de tubulure (A1) étant formé par le premier et le second élément de rupture (30, 40) du premier agencement rupteur (3, 4), au moins un des éléments de rupture (30, 40) étant déplaçable latéralement par rapport à l'autre élément de rupture (30, 40) pour agir sur un élément de fermeture (70) d'une tubulure (7) insérée dans le premier dispositif de réception de tubulure (A1).

3. Dispositif d'ouverture (1) selon la revendication 1 ou 2, **caractérisé en ce que** le second agencement rupteur (5) comprend un premier élément de rupture (50) et un second élément de rupture (51), le second dispositif de réception de tubulure (A2) étant formé par le premier et le second élément de rupture (50, 51) du second agencement rupteur (5), au moins l'un des premier et second éléments de rupture (50, 51) pouvant pivoter par rapport à l'autre élément de rupture (50, 51) pour agir sur un élément de fermeture (70) d'une tubulure (7) insérée dans le second dispositif de réception de tubulure (A2).

4. Dispositif d'ouverture (1) selon l'une des revendications 1 à 3, **caractérisé en ce que** le mécanisme d'ouverture (2) comprend un premier élément d'actionnement (3) et un second élément d'actionnement (4), le premier et le second élément d'actionnement (3, 4) étant mobiles latéralement l'un par rapport à l'autre pour actionner le premier et/ou le second agencement rupteur (3, 4, 5), l'agencement d'entraînement (6) pouvant venir en prise avec le premier et/ou le second élément d'actionnement (3, 4) pour entraîner le mécanisme d'ouverture (2).

5. Dispositif d'ouverture (1) selon la revendication 4, **caractérisé en ce qu'**un premier élément de rupture (30) du premier agencement rupteur (3, 4) est relié rigidement au premier élément d'actionnement (3) et un second élément de rupture (40) du premier agencement rupteur (3, 4) est relié rigidement au second élément d'actionnement (4).

6. Dispositif d'ouverture (1) selon la revendication 4 ou 5, **caractérisé en ce que** le second agencement rupteur (5) est couplé mécaniquement au premier et/ou au second élément d'actionnement (3, 4) de telle sorte qu'un premier élément de rupture (50) et/ou un second élément de rupture (51) du second agencement rupteur (5) peuvent pivoter en déplaçant latéralement le premier et/ou le second élément d'actionnement (3, 4).

7. Dispositif d'ouverture (1) selon la revendication 6, **caractérisé en ce que** le premier élément de rupture (50) du second agencement rupteur (5) est agencé de manière rotative sur le premier élément d'actionnement (3), et le second élément de rupture (51) du second agencement rupteur (5) est relié de manière pivotante au premier élément de rupture (50) du second agencement rupteur (5), en outre, le premier élément de rupture (50) du second agencement rupteur (5) étant couplé au second élément d'actionnement (4) et le second élément de rupture (51) étant couplé au premier élément d'actionnement (3) de telle sorte qu'un mouvement relatif du premier élément d'actionnement (3) et du second élément d'actionnement (4) l'un par rapport à l'autre provoque un pivotement du premier élément de rupture (50) et du second élément de rupture (51) du second agencement rupteur (5) l'un par rapport à l'autre.

8. Dispositif d'ouverture (1) selon l'une des revendications 4 à 7, **caractérisé en ce que** le dispositif d'entraînement comprend un élément d'entraînement (6) pouvant tourner autour d'un axe de rotation (R) et venir en prise avec au moins l'un parmi le premier et le second élément d'actionnement (3, 4).

9. Dispositif d'ouverture (1) selon la revendication 8, **caractérisé en ce que** l'élément d'entraînement (6) est constitué pour agir sur le mécanisme d'ouverture (2)
- dans la première position du mécanisme d'ouverture (2) pour actionner le premier agencement rupteur (3, 4) pour agir sur une tubulure (7) dans le premier dispositif de réception (A1),
- entre la première position et la seconde position pour déplacer le mécanisme d'ouverture (2) entre la première et la seconde position, et
- dans la seconde position du mécanisme d'ouverture (2) pour actionner le second agencement rupteur (5) pour agir sur une tubulure (7) dans le second dispositif de réception (A2).

10. Dispositif d'ouverture (1) selon la revendication 8 ou 9, **caractérisé en ce que** l'élément d'entraînement (6) vient en prise avec le premier élément d'actionnement (3), mais pas avec le second élément d'actionnement (4), pour déplacer le mécanisme d'ouverture (2) entre la première et la seconde position, le second élément d'actionnement (4) étant porté avec le premier élément d'actionnement (3) lors du déplacement du premier élément d'actionnement (3).

11. Dispositif d'ouverture (1) selon l'une des revendications 8 à 10, **caractérisé en ce que** l'élément d'entraînement (6) comprend deux broches (61, 62) en saillie par rapport à un corps (60) de l'élément d'entraînement (6) le long de l'axe de rotation (R) et ayant des longueurs différentes lorsqu'on les regarde le long de l'axe de rotation (R), une première des deux broches (61, 62) ayant une première longueur et étant constituée pour venir en prise avec à la fois le premier et le second élément d'actionnement (3, 4), tandis qu'une seconde des deux broches (61, 62) a une seconde longueur inférieure à la première longueur et est constituée pour venir en prise uniquement avec le premier élément d'actionnement (3).

12. Dispositif d'ouverture (1) selon l'une des revendications précédentes, **caractérisé en ce que** le dispositif d'entraînement (6) est constitué pour entraîner de manière électromécanique le mécanisme d'ouverture (2) .

13. Dispositif d'ouverture (1) selon l'une des revendications précédentes, **caractérisé en ce que** la tubulure (7) est insérable dans le premier dispositif de réception de tubulure (A1) ou dans le second dispositif de réception de tubulure (A2) dans une direction d'insertion (E), le mécanisme d'ouverture (2) étant déplaçable par rapport au boîtier (10) le long d'une direction de déplacement (V) transversale à la direction d'insertion (E).
